Europäisches Patentamt

European Patent Office

Office européen des brevets

(11) Publication number: **0 409 279 A2**

# EUROPEAN PATENT APPLICATION

(21) Application number: **90113980.8**

(22) Date of filing: **20.07.90**

(51) Int. Cl.⁵: **A61K 9/20**

(30) Priority: **21.07.89 JP 189213/89**

(43) Date of publication of application:
**23.01.91 Bulletin 91/04**

(84) Designated Contracting States:
**BE CH DE ES FR GB IT LI NL SE**

(71) Applicant: **SS PHARMACEUTICAL CO., LTD.**
**2-12-4 Nihonbashi Hama-cho Chuo-ku**
**Tokyo(JP)**

(72) Inventor: **Kanbe, Hideyoshi**
**2-21-8, Mama**
**Ichikawa-shi, Chiba(JP)**
Inventor: **Ohsugi, Tomohiko**
**653-32, Obukuro**
**Narita-shi, Chiba(JP)**
Inventor: **Yokoyama, Toshio**
**7-19-5-109, Machiya, Arakawa-ku**
**Tokyo(JP)**
Inventor: **Iwasa, Akira**
**886-16-1, Shikawatashi**
**Yotsukaido-shi, Chiba(JP)**

(74) Representative: **Wächtershäuser, Günter, Dr.**
**Tal 29**
**D-8000 München 2(DE)**

(54) **Troche composition.**

(57) A troche composition comprising xylitol as a filler and gelatin as a binder, in combination, is disclosed. The troche composition, when administered, is not felt coarse or powdery, but gives a favorable feeling.

EP 0 409 279 A2

# TROCHE COMPOSITION

## BACKGROUND OF THE INVENTION

Field of the Invention :

This invention relates to a troche composition which imparts a favorable oral sensation.

Description of the Background Art :

Troches are designed so as to be convenient for buccal application and also to gradually dissolve and disintegrate from the surface for ensuring gradual discharge of the pharmaceutical component, while maintaining the activity of its pharmaceutical component for a long period of time. For this reason, sugars, such as purified sucrose, lactose, glucose, fructose, maltose, mannitol, or the like are used as excipients; and binders such as hydroxypropylcellulose, polyvinylpyrrolidone, potato starch, acacia, tragacanth, and the like, and lubricants such as talc, stearic acid, magnesium stearate, calcium stearate, polyoxyethylenemonostearate, polyoxyethylenelauryl alcohol ether, and the like are used in a larger amount than in other common tablets. In addition, correctives or corrigents are added.

These conventional troches, however, impart coarse and powdery feelings, and thus are not favorably accepted by patients in need thereof.

In view of this situation, the present inventors have undertaken extensive studies in order to develop troches which are not coarse or powdery, but give a favorable feeling when administered, and, as a result, found that such an objective could be resolved by the combined use of xylitol and gelatin.

## SUMMARY OF THE INVENTION

Accordingly, an object of this invention is to provide a troche composition comprising xylitol as a filler and gelatin as a binder.

Other objects, features and advantages of the invention will hereinafter become more readily apparent from the following description.

## DETAILED DESCRIPTION OF THE INVENTION AND PREFERRED EMBODIMENTS

The amount of xylitol used in the composition of the present invention is 25 to 99% by weight, preferably 50 to 99% by weight, of the total amount of the troche composition.

Gelatin to be used in the composition of the present invention is not specifically limited so long as the same has been prepared for pharmaceutical use. Usually, preferable gelatins are purified gelatin having a molecular weight of 7,000 to 100,000 which is obtained from collagen through the treatment by an acid or an alkali followed by hydrolysis by heating, succinated gelatin produced by the association or polymerization of said purified gelatin with succinic acid or formalin, and low molecular weight water- soluble gelatin of a molecular weight of 3,000 to 15,000 prepared by hydrolysis of gelatins.

A preferable amount of gelatin to be incorporated into the troche composition of the present invention is 0.1 to 20, more preferably 0.2 to 8, parts by weight per 100 parts by weight of xylitol.

In the present invention, the combined use of xylitol and gelatin provides troches which are free from a coarse and powdery feeling, but impart a slippery, favorable feeling to the tongue. Absence of either of the two components does not give the effect of the present invention.

The use of a sugar alcohol other than xylitol, as a filler, e.g. sorbitol, D-mannitol; or a sugar which is conventionally used as a filler of troches, e.g. refined white sugar, lactose, glucose, fructose, maltose or the like, does not give the intended effect even if they are used in combination with gelatin.

In the same way, an addition of agar, acacia powder, or the like, which gives a viscous liquid when dissolved in hot water, to xylitol or to other conventional binder such as hydroxypropylcellulose, polyvinylpyrrolidone, potato starch, or the like, does not produce the effect which can be obtained by the combined use of xylitol and gelatin.

Besides the above two essential components, other medicinal components such as preservatives, antiseptics, antibiotics, antiphlogistics, local anesthetics, antitussives, expectorants, astringents, tooth cleanser, and the like; as well as components such as lublicants, correctives or corrigents, may be optionally added to the extent that the effect of the present invention is not impaired.

The wet molding or compression molding method which is conventionally used for the preparation of troches can be used for the preparation of troches of the present invention.

When the wet molding method is used, the troche tablets can be prepared, for example, by mixing finely pulverized xylitol, pharmaceutically active components and other optional components, e.g. a corrective, a corrigent, or the like, kneading the mixture together with an aqueous solution of gelatin to produce plastic wet lumps, molding the plastic material into a uniform shape, then drying it.

For compression molding, either an indirect or direct tabletting method can be used. The indirect method comprises granulating a homogeneous mixture of pharmaceutically active components, xylitol, gelatin, and, optionally, a corrective, corrigent, or the like, adding a lubricant to the granule, and compression-molding the mixture. In a direct tabletting method, a homogeneous mixture of pharmaceutically active components, xylitol, gelatin, and other optional components, e.g. a lubricant, a corrective, a corrigent, or the like is directly compression-molded. Alternatively, granules prepared from the components other than pharmaceutically active components is homogeneously mixed with pharmaceutically active components, and the mixture is compress-molded.

Other features of the invention will become apparent in the course of the following description of the exemplary embodiments which are given for illustration of the invention and are not intended to be limiting thereof.

## EXAMPLES

| Example 1 | |
|---|---|
| Lysozyme chloride | 5 g |
| Xylitol (Xylit P, product of Towa Kagaku Kogyo Co.) | 978 g |
| Alkali-treated gelatin (SE1, product of Nippi Co.) | 4 g |
| Stearic acid | 10 g |
| Magnesium stearate | 2 g |
| Talc | 1 g |
| Total | 1,000 g |

Lysozyme chloride and xylitol were mixed in a Henshel mixer (20 liters; manufactured by Mitsui-Miike Manufacturing Co.), and 20 g of a 20 wt.% aqueous solution of gelatin was added to the mixture. The whole mixture was kneaded, dried at 50 °C, and passed through a 20 mesh sieve. After an addition of lubricants (straeric acid, magnesium stearate, and talc), the mixture was made into troches of a disk shape with a 15 mm diameter.

3

| Example 2 | |
|---|---|
| Lysozyme chloride | 5 g |
| Xylitol (Xylit P, product of Towa Kagaku Kogyo Co.) | 974 g |
| Alkali-treated gelatin (SE1, product of Nippi Co.) | 8 g |
| Stearic acid | 10 g |
| Magnesium stearate | 2 g |
| Talc | 1 g |
| Total | 1,000 g |

Troches of a disk shape with a 15 mm diameter were prepared in the same manner as in Example 1, except that 40 g, instead of 20 g in Example 1, of a 20 wt.% aqueous solution of gelatin was used.

| Example 3 | |
|---|---|
| Lysozyme chloride | 5 g |
| Xylitol (Xylit P, product of Towa Kagaku Kogyo Co.) | 970 g |
| Alkali-treated gelatin (SE1, product of Nippi Co.) | 12 g |
| Stearic acid | 10 g |
| Magnesium stearate | 2 g |
| Talc | 1 g |
| Total | 1,000 g |

Troches of a disk shape with a 15 mm diameter were prepared in the same manner as in Example 1, except that 60 g, instead of 20 g in Example 1, of a 20 wt.% aqueous solution of gelatin was used.

Example 4

Troches of a disk shape with a 15 mm diameter were prepared in the same manner as in Example 2, except that, instead of alkali-treated gelatin, the same amount of acid-treated gelatin (CP-326, product of Miyagi Kagaku Kogyo Co.) was used.

Example 5

Troches of a disk shape with a 15 mm diameter were prepared in the same manner as in Example 2, except that, instead of alkali-treated gelatin, the same amount of succinated gelatin (CP-366, product of Miyagi Kagaku Kogyo Co.) was used.

Example 6

Troches of a disk shape with a 15 mm diameter were prepared in the same manner as in Example 2, except that, instead of alkali-treated gelatin, the same amount of water-soluble gelatin (TG, product of Miyagi Kagaku Kogyo Co.) was used.

| Comparative Example 1 | |
|---|---|
| Lysozyme chloride | 5 g |
| Xylitol (Xylit P, product of Towa Kagaku Kogyo Co.) | 982 g |
| Stearic acid | 10 g |
| Magnesium stearate | 2 g |
| Talc | 1 g |
| Total | 1,000 g |

Troches of a disk shape with a 15 mm diameter were prepared in the same manner as in Example 1, except that, instead of the gelatin solution, 80 g of purified water was used.

Comparative Example 2

Troches of a disk shape with a 15 mm diameter were prepared in the same manner as in Example 2, except that, instead of xylitol, the same amount of purified sucrose was used.

Comparative Example 3

Troches of a disk shape with a 15 mm diameter were prepared in the same manner as in Example 2, except that, instead of xylitol, the same amount of D-mannitol was used.

Comparative Example 4

Troches of a disk shape with a 15 mm diameter were prepared in the same manner as in Example 2, except that, instead of xylitol, the same amount of sorbitol was used.

Comparative Example 5

Troches of a disk shape with a 15 mm diameter were prepared in the same manner as in Example 2, except that, instead of xylitol, the same amount of lactose was used.

Comparative Example 6

Troches of a disk shape with a 15 mm diameter were prepared in the same manner as in Example 2, except that, instead of the aqueous solution of gelatin, 80 g of a 10 wt.% aqueous solution of hydroxypropylcellulose (HPC-L, product of Nippon Soda Co.) was used.

Comparative Example 7

Troches of a disk shape with a 15 mm diameter were prepared in the same manner as in Example 2, except that, instead of the aqueous solution of gelatin, 80 g of a 10 wt.% aqueous solution of agar was used.

Comparative Example 8

Troches of a disk shape with a 15 mm diameter were prepared in the same manner as in Example 2, except that, instead of the aqueous solution of gelatin, 80 g of a 10 wt.% aqueous solution of acacia was used.

EP 0 409 279 A2

Comparative Example 9

Troches of a disk shape with a 15 mm diameter were prepared in the same manner as in Example 2, except that, instead of the aqueous solution of gelatin, 80 g of a 10 wt.% aqueous solution of potato starch was used.

Comparative Example 10

Troches of a disk shape with a 15 mm diameter were prepared in the same manner as in Example 2, except that, instead of the aqueous solution of gelatin, 80 g of a 10 wt.% aqueous solution of polyvinylpyrrolidone (PVP-K90, product of BASF) was used.

Test Example

Feelings upon the administration of troches prepared in Examples 1-6 and Comparative Examples 1-10 were compared by a panel of 6 persons. It was confirmed that the troches of Examples 1-6 were all felt slippery and gave favorable feelings without no powdery sensation. By contrast, all troches of Comparative Examples 1-10 were felt powdery and gave objectionable feelings upon administration.

As illustrated above, the invention provides troches which are not coarse or powdery, but give a favorable slippery feeling when administered.

Obviously, numerous modifications and variations of the present invention are possible in light of the above teachings. It is therefore to be understood that within the scope of the appended claims, the invention may be practiced otherwise than as specifically described herein.

**Claims**

1. A troche composition comprising xylitol as a filler and gelatin as a binder.
2. A troche composition according to Claim 1, wherein the amount of gelatin is 0.1 to 20 parts by weight per 100 parts by weight of xylitol.